# EUROPÄISCHE PATENTANMELDUNG

(11) **EP 1 066 806 A1**
(43) Veröffentlichungstag der Anmeldung: **10.01.2001**
(21) Anmeldenummer: 00250225.0
(22) Anmeldetag: 08.07.2000
(51) Int. Cl.: A61F 2/34

(54) **Pfanne für eine Hüftgelenkendoprothese**

(30) Priorität: 08.07.1999 DE 29911853 U
(71) Anmelder: Biomet Merck Deutschland GmbH, 14167 Berlin (DE)
(72) Erfinder: Calisse, Jorge, 10785 Berlin (DE)
(74) Vertreter: Gross, Felix, Dr.

(57) **Zusammenfassung**

Pfanne für eine Hüftgelenkendoprothese, mit einer Außenschale (2), einer Innenschale (20) in der Außenschale (2), mit einem innen an der Außenschale (2) mindestens abschnittweise umlaufenden Stützansatz (10), und mit einer außen an der Innenschale (20) mindestens abschnittweise umlaufenden federnden Stützlippe (30), die schräg abwärts nach außen gerichtet ist und den Stützansatz (10) hintergreift, wobei die Stützfläche (12) des Stützansatzes (10), gegen welche die Stützlippe (30) anliegt, nach außen ansteigt und die Stützlippe (30) gegen das Pfannenzentrum drückt.

## Beschreibung

Die Erfindung betrifft eine Pfanne für eine Hüftgelenkendoprothese, mit einer Außenschale und einer Innenschale in der Außenschale, mit einem innen an der Außenschale mindestens abschnittweise umlaufenden Stützansatz, und mit einer außen an der Innenschale mindestens abschnittweise umlaufenden federnden Stützlippe, die schräg abwärts nach außen gerichtet ist und den Stützansatz hintergreift.

Eine derartige Pfanne wird beispielsweise von der Anmelderin vermarktet, bei welcher die Stützfläche des ringförmigen Stützansatzes paralle zur Ebene des Pfannenrandes verläuft und mit der Stützlippe der Innenschale eine Schnappverbindung eingeht, welche die Innenschale sicher in der Außenschale hält. Im implantierten Zustand werden jedoch die durch die zwischen Pfanne und Gelenkkugel des Oberschenkelteils wirkenden Kräfte stark beansprucht. Dies hat zur Folge, daß die Innenschale bei längerer Nutzungsdauer stärker in den Hohlraum der Außenpfanne hineingedrückt wird und auch dabei verformt. Die Verformung und Lageänderung der Innenschale hat zur Folge, daß die Stützlippe sich von der Stützfläche abhebt, so daß die Stützfläche und die Stützlippe nicht mehr zur Lagesicherung der Innenschalebeiträgt, so daß die Gefahr einer Lockerung der Innenschale gegeben ist. Nachteilig ist außerdem, daß die aufgrund unvermeidlichen Abriebs zwischen Außenschale und Innenschale entstehenden Partikel sich durch den dann vorhandenen Freiraum zwischen Stützfläche und Stützlippe hindurch in das menschliche umliegende Gewebe bewegen können und dort Ursache von Entzündungen sind.

Aufgabe der Erfindung ist es daher, eine Pfanne der eingangs genannten Art derart weiterzubilden, daß auch nach der Implantation langzeitig eine sichere Lagefixierung der Innenschale in einfacher Weise gewährleistet ist.

Diese Aufgabe wird bei der Pfanne der eingangs genannten Art erfindungsgemäß dadurch gelöst, daß die Stützfläche des Stützansatzes, gegen welche die Stützlippe anliegt, nach außen ansteigt und die Stützlippe gegen das Pfannenzentrum drückt.

Die Vorteile der Erfindung liegen insbesondere darin, daß die Stützlippe gegen die schräg nach außen ansteigende Stützfläche anliegt, wenn die Innenschale in die Außenschaule eingesetzt ist. Die schräg nach außen ansteigende Stützfläche hat zur Folge, daß die Stützlippe eine gegen das Zentrum der Pfanne, also nach innen gerichtete Kraftkomponente erfährt, wenn die Stützlippe zur Anlage an die schräge Stützfläche kommt. Wenn dann unter der Langzeitbelastung die Innenschale weiter in die Außenschale hineingedrückt wird oder sich entsprechend verformt, so erhält die unter Vorspannung stehende Stützlippe Freiraum, und kann sich - unter ihrer Vorspannung - mit ihrem freien Ende längs der schrägen Stützfläche nach außen bewegen, bleibt dabei jedoch in Berührungskontakt mit der schrägen Stützfläche. Stützfläche und Stützlippe tragen daher auch dann noch zur Lagefixierung der Innenschale bei, wenn nach der Implantation die Innenschale -durch die Langzeitbelastung - stärker in die Außenschale hineinsackt. Die zwischen Innenschale und Außenschale durch ihre relative Mikrobewegung erzeugten Abrieb-Partikel verbleiben außerdem in der Pfanne, sie können die Pfanne nicht durch einen Freiraum zwischen Stützlippe und Stützfläche verlassen. Die Gefahr, daß diese Abriebpartikel das umliegende menschliche Gewebe stören, ist dadurch beseitigt.

Gemäß einer weiteren bevorzugten Ausführungsform der Erfindung steigt die Stützfläche des Stützansatzes nach außen hin stufenförmig an. Die Stufen können sich mit ihren Kanten in das freie Ende der Stützlippe eingraben und - unter der Wirkung der Mikrobewegungen - das freie Ende der Stützlippe komplementär ausformen. Dadurch wird zwischen Stützfläche und Stützlippe ein Formschluß erzeugt, der die Fixierung der Innenschale in der Außenschale verbessert. Die Stufen besitzen bevorzugt eine Höhe, die vom Abstand zum Pfannenzentrum und außerdem von der Ausrichtung der Stützlippe abhängt.

Gemäß einer weiteren bevorzugten Ausführungsform der Erfindung befindet sich über der Stützfläche des Stützansatzes ein Hinterschnitt, der so groß bemessen ist, daß die Stützlippe einen ausreichenden Freiraum hat, um sich radial auswärts längs der schrägen Stützfläche zu bewegen, wenn die Innenschale stärker in die Außenschale hineingedrückt wird. Außerdem kann sich in diesem Hinterschnitt der Abrieb aufsammeln, der zwischen Innenschale und Außenschale durch die ständige Gelenkbewegung erzeugt wird.

Vorteilhafterweise läuft die Stützfläche und die Stützlippe vollständig ringförmig um Innenschale bzw. Außenschale herum, so daß eine Stützverbindung oder Schnappverbindung um 360° vorliegt, die Stützkräfte also gleichförmige zwischen Außenschale und Innenschale wirken und ein ringförmiger gleichmäßiger Berührungskontakt zwischen Stützfläche und Stützlippe gebildet ist.

Gemäß einer weiteren bevorzugten Ausführungsform der Erfindung ist der Stützansatz am Rand der Außenpfanne angeordnet, bevorzugt schließt er eben mit einer ebenen Randfläche ab. Bevorzugt ist das freie Ende der Stützlippe im Querschnitt abgerundet oder angefast, damit die Stützlippe sich auch bei gestufter Stützfläche frei über die Stufen hinwegbewegen kann.

Vorteilhafte Weiterbildungen der Erfindung sind durch die Merkmale der Unteransprüche gekennzeichnet.

Im folgenden wird ein Ausführungsbeispiel anhand der Zeichnung näher erläutert. Es zeigen:
- Fig. 1: einen Querschnitt durch eine Pfanne: und
- Fig. 2: einen vergrößerten Querschnitt der Pfanne im Bereich der Stützverbindung zwischen Innenschale und Außenschale.

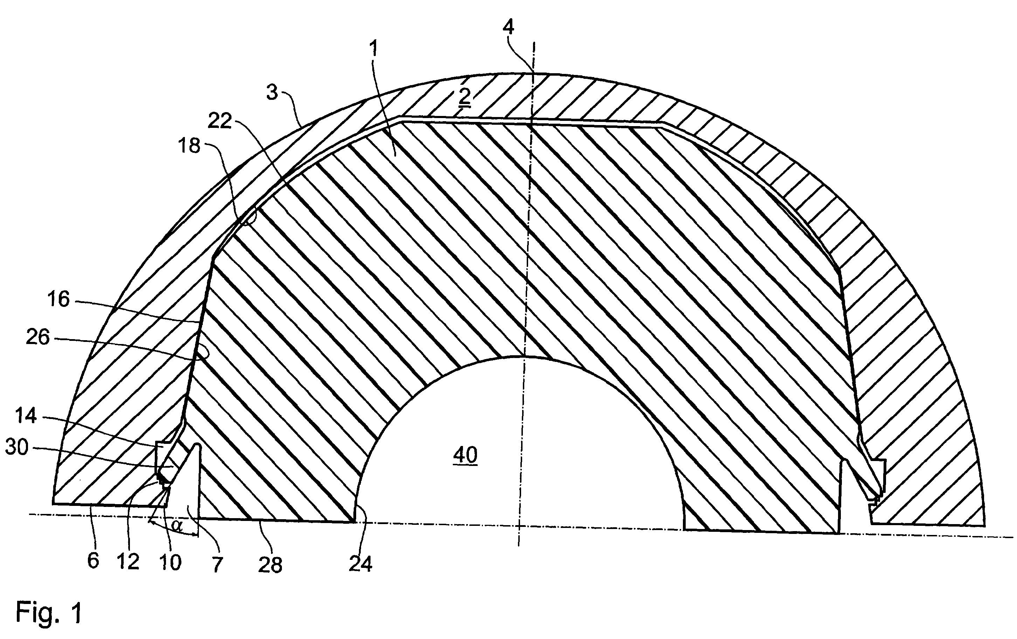

Figur 1 zeigt einen Querschnitt durch die Pfanne einer Hüftgelenkendoprothese. Eine Außenschale 2, bevorzugt aus Metall, besitzt in der dargestellten Ausführungsform eine Außenfläche 3, die im wesentlichen rotationssymmetrisch zu einer Rotationsachse 1 verläuft und einen Kugelabschnitt, nahezu einen Halbkugelabschnitt bildet. Die Außenfläche 3 besitzt an der Stelle, wo die Rotationsachse 1 die Außenfläche durchstößt, einen Pol 4, sie erstreckt sich bis zu einem Pfannenrand 6, der in einer Ebene senkrecht zur Rotationsachse 1 kreisringförmig verläuft.

Eine kreisscheibenförmige Öffnung 7 begrenzt den Innenraum 8 der Außenschale 2, in den eine Innenschale 20 aus Kunststoffmaterial oder Metall eingesetzt ist. Die Außenfläche 22 der Innenschale 20 ist an die Innenfläche 18 der Außenschale 2 angepaßt. Die Innenfläche 24 der Innenschale 20 bildet einen halbkugelförmigen Hohlraum 40, der zur Aufnahme des Kugelkopfes eines entsprechenden prothetischen Oberschenkelteiles dient.

Die Innenschale 20 besitzt ebenfalls einen kreisringförmigen Rand 28, der etwa in der Ebene des Pfannenrandes 6 der Außenschale senkrecht zur Rotationsachse 1 umläuft.

Die Innenschale 20 und die Außenschale 2 besitzen eine konusförmige Passung 16, 26, die einerseits an der Innenfläche 16 der Außenschale 2, andererseits an der Außenfläche 26 der Innenschale 20 ausgebildet ist. Diese konusförmige Passung hat zur Folge, daß die Innenschale 20 bei der im eingebauten Zustand vorhandenen Belastung in die Außenschale 2 hineingedrückt wird und sich dort verklemmt.

Innen an der Außenschale 2 ist ein sich gegen die Rotationsachse 1 erstreckender Stützansatz 10 vorgesehen, der ringförmig um die Rotationsachse 1 umläuft und eine nach außen ansteigende Stützfläche 12 besitzt - in der dargestellten Ausführungsform in mehreren Stufen unterschiedlicher Höhe zu einer Basisfläche 13a ansteigt - über den Stufen 13 - einen Hinterschnitt 14 radial begrenzt.

Außen an der Innenschale 20 ist eine ringförmige federnde Stützlippe 30 angeformt, die schräg abwärts nach außen gerichtet ist und deren freies Ende 32 abgerundet oder angefast ist. Der Winkel , den die Stützlippe 30 gegen eine vertikale Fläche einschließt, liegt in der dargestellten Ausführungsform zwischen etwa 20° und 50°.

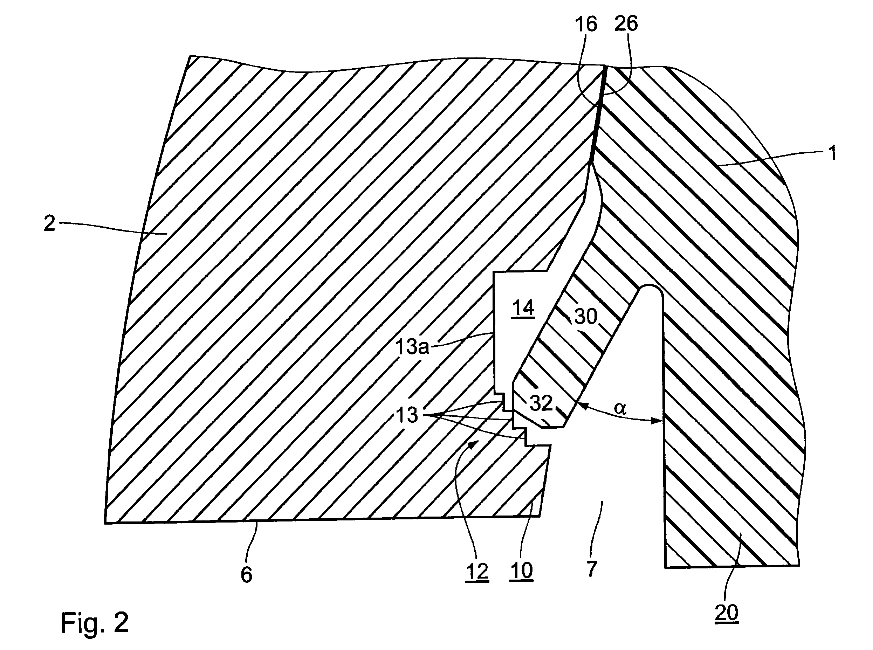

Wie insbesondere in Figur 2 dargestellt ist, liegt die Stützlippe 30 der Innenschale 20 mit ihrem freien Ende gegen die Stützfläche 12 des Stützansatzes 10 an. Die Länge der Stützlippe 30 und ihre Form sind so bemessen, daß die Stützlippe 30, wenn die Konuspassung 16, 26 in eine Klemmpassung übergeht, unter der radial nach außen ansteigenden Stützfläche 12 ein gegen das Pfannenzentrum oder die Rotationsachse 1 gerichtete Kraft oder Vorspannung erfährt.

Unter dieser Kraft wird die federnde Stützlippe 30 nach innen vorgespannt und bleibt in diesem Zustand, solange die Position der Innenschale sich nicht verändert. Wenn im implantierten Zustand unter der Dauerbeanspruchung die Innenschale 20 dann weiter nach innen sackt, und sich unter der starken ständigen Walkbeanspruchung auch verformt, so hat dies zur Folge, daß die vorgespannte Stützlippe 30 längs der schräg ansteigenden Stützfläche 12 nach oben läuft und sich dabei auch stärker - unter der eigenen Elastizität - nach außen ausstellt. Dabei trägt die Stützverbindung zwischen Stützlippe 30 und Stützfläche 12 dazu bei, die dann von der Innenschale 20 eingenommene Lage auch weiterhin in der Außenschale 2 zu fixieren.

Der Stützansatz 10 ist am Rand 6 der Außenpfanne 2 angeordnet und verläuft - mit derselben Randebene radial gegen die Rotationsachse.

Das freie Ende 32 der Stützlippe 30 ist abgerundet oder angefast, um zu verhindern, daß dieses freie Ende 32 der Stützlippe 30 sich an den Stufen 13 der Stützfläche 12 verkantet.

## Patentansprüche

1. Pfanne für eine Hüftgelenkendoprothese, mit einer Außenschale, einer Innenschale in der Außenschale, mit einem innen an der Außenschale mindestens abschnittweise umlaufenden Stützansatz, und mit einer außen an der Innenschale mindestens abschnittweise umlaufenden federnden Stützlippe, die schräg abwärts nach außen gerichtet ist und den Stützansatz hintergreift,
dadurch gekennzeichnet, daß die Stützfläche (12) des Stützansatzes (10), gegen welche die Stützlippe (30) anliegt, nach außen ansteigt und die Stützlippe (30) gegen das Pfannenzentrum drückt.

2. Pfanne nach Anspruch 1,
dadurch gekennzeichnet, daß die Stützfläche (12) nach außen in mehreren Stufen (13) ansteigt.

3. Pfanne nach Anspruch 2,
dadurch gekennzeichnet, daß die Stufen (13) der Stützfläche (12) alle dieselbe Höhe aufweisen.

4. Pfanne nach Anspruch 2,
dadurch gekennzeichnet, daß die Stufen (13) der Stützfläche (12) verschiedene Höhen aufweisen.

5. Pfanne nach Anspruch 4,
dadurch gekennzeichnet, daß die Höhe der Stufe (13) eine Funktion des Abstandes ist, den die Stufe (13) zum Pfannenzentrum hat.

6. Pfanne nach einem der vorstehenden Ansprüche,
dadurch gekennzeichnet, daß über dem Stützansatz (10) ein Hinterschnitt (14) in der Außenschale (2) vorgesehen ist.

7. Pfanne nach einem der vorstehenden Ansprüche,
dadurch gekennzeichnet, daß der Stützansatz (10) ringförmig an der Innenfläche der Außenschale (2) um 360° umläuft.

8. Pfanne nach einem der vorstehenden Ansprüche,
dadurch gekennzeichnet, daß die federnde Stützlippe (30) an der Außenfläche der Innenschale (20) ringförmig um 360° umläuft.

9. Pfanne nach einem der vorstehenden Ansprüche,
dadurch gekennzeichnet, daß der Stützansatz (10) am Rand (6) der Außenpfanne (2) angeordnet ist.

10. Pfanne nach einem der vorstehenden Ansprüche,
dadurch gekennzeichnet daß der Rand (6) der Außenpfanne (2) eben ist und stufenlos in den Stützansatz (10) übergeht.

11. Pfanne nach einem der vorstehenden Ansprüche,
dadurch gekennzeichnet, daß die Innenschale (20) und die Außenschale (2) über dem Stützansatz (10) und der Stützlippe (30) eine konusförmige Passung (16, 28) bilden.

12. Pfanne nach einem der vorstehenden Ansprüche,
dadurch gekennzeichnet, daß die Stützlippe (30) an ihrem freien Ende abgerundet oder angefast ist.

13. Pfanne nach einem der vorstehenden Ansprüche,
dadurch gekennzeichnet, daß die Innenschale (20) aus Kunststoff oder Metall besteht.
